# EUROPEAN PATENT SPECIFICATION

(11) **EP 2 227 195 B1**
(45) Date of publication and mention of the grant of the patent: **27.08.2014**
(21) Application number: 08852437.6
(22) Date of filing: 20.11.2008
(51) Int. Cl.: A61F 5/56

(54) **METHODS AND KITS FOR MAKING FLEXIBLE DENTAL GUARDS**
VERFAHREN UND KITS ZUR HERSTELLUNG VON FLEXIBLEN ZAHNSCHUTZVORRICHTUNGEN
PROCÉDÉS ET KITS POUR LA FABRICATION DE PROTECTIONS DENTAIRES FLEXIBLES

(30) Priority: 20.11.2007 US 986197
(43) Date of publication of application: 15.09.2010
(73) Proprietor: DENTSPLY International Inc., York, PA 17405-0872 (US)
(72) Inventor: SUN, Benjamin, Jiemin, York PA 17402 (US); SHAFFER, Scott, Earl, Jacobus PA 17407 (US); YOUNG, Andrew, Matthias, Dallastown PA 17313 (US)
(74) Representative: Hartz, Nikolai
(86) International application number: PCT/US2008/012971
(87) International publication number: WO 2009/067236

(56) References cited:
- WO-A-2007/120495
- US-A1- 2004 224 283
- US-A1- 2006 121 407

## Description

### BACKGROUND OF THE INVENTION

### Field of the Invention

The present invention relates generally to a method and a kit for making dental guards for protecting human teeth against the effects of grinding and clenching, a condition commonly referred to as bruxism according to the definition in the preamble of claim 1. Such a device is known from US-A-2004/224 283.

### Brief Description of the Related Art

Dental practitioners commonly prescribe dental night guards or splints to patients who grind and clench their teeth. This is a particularly chronic problem for many patients during sleep time. In practice, the night guard is placed over a patient's upper or lower teeth so that it acts as a protective sleeve. Typically, the night guard is placed over the patient's upper dental arch. The night guard prevents the upper and lower tooth surfaces from directly contacting each other. In this manner, the night guard prevents serious tooth damage that can result from tooth grinding and clenching.

In conventional procedures for making a dental night guard; a patient will make multiple visits to the dentist. In the first visit, the dentist takes an initial impression of the patient's dental anatomy. The impression material is normally prepared from two paste components. At least one of the paste components contain an elastomeric material such as vinyl terminated polysiloxanes capable of undergoing addition polymerization. Once the pastes are mixed together, they start to harden and form a rubbery material. The dentist dispenses the impression material into a bite tray and inserts the tray into a patient's mouth. The patient bites down on the impression material in the tray. Then, the tray is removed from the mouth and the impression material is allowed to cure and harden. A negative impression of the teeth is formed in the impression material.

The hardened impressions are sent to a dental laboratory that will fabricate the night guard. The dental technician, at the laboratory, prepares a cast (or model) by pouring dental plaster or stone into the hardened impression. This results in a finished plaster model having a shaped surface closely matching the patient's dental anatomy. Alternatively, if the dentist wishes, he or she can prepare the plaster models in their office and send the models to the laboratory.

The night guard is now ready to be fabricated using a vacuum thermoforming machine such as, for example, the Essix® Machine, available from Dentsply Raintree Essix (Metairie, LA). Following conventional manufacturing techniques, the plaster dental model is placed on the vacuum plate of the machine. A hard sheet of thermoplastic resin is placed in the machine frame and positioned over the dental model. The heating element is swung over the plastic sheet and the sheet is heated until it begins to slightly sag. Then, the vacuum is turned on and the heated plastic sheet is lowered over the model. As the heated plastic melts onto the model, a probe is used to guide the molten plastic into the interproximal undercuts of the model. This ensures that the model is covered completely with the molten plastic. After this thermoforming step, the molten plastic should be cooled immediately. A refrigerant coolant such as Freeze Spray™ (Dentsply) or other cooling material can be sprayed onto the plastic. This causes the plastic to rapidly cool and shrink so that the plastic material fits more tightly around the cast. Upon completing this cooling step, the night guard is essentially fabricated and it just needs to be removed from the cast. To remove the night guard from the dental cast, the technician first uses scissors to trim away any excess material. Next, an electric knife is used to cut through the heel of the plastic enclosed cast. This helps prevent the cast from breaking as the night guard is removed. After the night guard has been removed, it is trimmed in detail to produce a finished appliance. If the technician wishes, the night guard can be placed back onto the cast, and the technician can swipe a butane torch around the night guard's edges to smooth out any rough spots.

The laboratory sends the finished night guard back to the dentist. At the second office visit, the dentist checks the occlusal fit of the night guard. And, if satisfactory, the dentist gives the night guard to the patient to take home and wear. The above-described techniques for making night guards are generally effective, although there are some drawbacks. For example, the finished night guard may not sit well in a patient's mouth. The night guard may not have good occlusal fit or marginal contacts making it prone to dislodge during sleep. Additionally, the finished night guard may have relatively poor durability and mechanical strength depending upon the materials used to make the night guard. If the night guard has poor wear-resistance, there is a risk that the plastic material will wear away over time and it will not act as barrier against tooth grinding and clenching. Moreover, the laboratory procedures used to make conventional night guards can be time-consuming and costly. The patient must make multiple office visits to the dentist in order to be fitted with conventional night guards. In view of these drawbacks, there have been some attempts in the dental industry to make new dental night guards using different plastic materials and techniques.

For example, Yousif, US Patent 5,103,838 discloses a night guard having a soft layer bonded to a hard layer. The soft layer makes contact with the patient's upper set of teeth while the hard layer forms the exterior of the night guard. To make the night guard, a wax flasking and boiling-out process is used to dispose a hard acrylic layer over a model of the patient's teeth. The model is also used to create the soft layer. The model and soft layer are positioned in one-half of the flask and the unfinished, hard exterior layer is positioned in the other half of the flask. A monomer or bonding material is placed on the interior surface of the hard, exterior layer and the two halves of the flask are joined together. The monomer bonds the hard, exterior layer with the soft layer. The night guard is cured and removed from the flask. In a second method, a sheet of soft material is inserted into a vacuum thermoforming machine and melted. A sheet of hard material is melted over the soft material.

Tregillis, US Patent 5,338,190 discloses a dental splint or night guard for treating bruxism. A wax flasking and boiling-out process is used to make the appliance which includes heat-cured methyl methacrylate and ethyl acrylate materials. The heat-cured methyl methacrylate covers the occlusal surface of the teeth, while the heat-cured ethyl acrylate covers the buccal side and lingual side of the teeth and may extend onto the gum tissue.

Sullivan, US Patent 6,241,518 discloses a bite guard for preventing the grinding of the upper and lower teeth of a person wearing braces. The bite guard generally includes a U-shaped base and plurality of hooks adapted to attach the bite guard to the arch wires of the braces. The bite guard is a single piece structure made by injecting a soft, rubber-like material into a mold and allowing the material to set.

Yoshida, US Patent 6,302,110 discloses a dental protector device for protecting against bruxism. The device generally has a U-shaped structure for conforming to the dental arch. The device includes a protective part adapted to cover the occlusal faces of the teeth and a contiguous engaging part adapted to cover the posterior surfaces of the teeth. The protective part is a binary layer structure consisting of a lower stratum which rests directly on the teeth and a planar upper stratum which is abutted against the occlusal faces of the upper teeth. The lower stratum of the protective part and engaging part are made from a thermoplastic resin having a softening temperature (e.g., 50-90°C) higher than the human body temperature but lower than the boiling temperature of water. These resins include ethylene-vinyl acetate copolymer, polyurethane, silicone, and poly(vinyl acetate). The upper stratum is made of a material which does not soften at the softening temperature, for example, silicone rubber or elastomers. The process for making the night guard involves immersing the protector device in hot water to warm the material. The warmed protector device is inserted in the mouth, and the patient bites down to make an impression mark. The protector device is then cooled to a temperature below the softening temperature of the material. The finished protector includes an impression mark conforming to the patient's teeth.

Sun et al., US Patent Application Publication US 2004/0224283 discloses a polymerizable composition which can be light-cured to form a dental splint or nightguard. The polymerizable composition does not contain any filler material. The polymerized composition, at 37°C, is described as having flexural modulus of less than 250,000 psi and flexural strength of less than 7,000 psi. In one example, the polymerizable material is shaped over a plaster cast of a patient's teeth and the cast is placed in a light-curing unit. In another instance, the polymerizable material is partially-cured in the mouth of a patient using a handheld curing light.

Liddle et al., US Patent Application Publication US 2005/0034733 discloses an interim dental guard which can be worn by a patient while waiting for a permanent night guard to be made and fitted. The method includes heating the device in a pre-formed condition to a first temperature in order to soften the device. This is placed in a patient's mouth and molded around the teeth to form an impression thereof. The device is cooled to a second temperature at which point the tooth impression is retained. The device is made of a thermoplastic material that is moldable when heated to a temperature above body temperature, preferably between 45 and 75°C. The device is stable enough so that it retains its shape when the temperature decreases to below 37°C (for example, normal body temperature). The thermoplastic material may include a color additive which changes the color of the material when it is heated to the softening point.

Although some night guards described in the foregoing patents have some desirable properties, there is still a need for developing new night guards to protect against the effects of bruxism. Particularly, there is a need for night guards having improved comfort, stability, and fit. The night guard should be flexible so that it conforms easily to a patient's teeth and is comfortable to wear. The patient should be able to insert and remove the night guard easily. At the same time, the night guard should be made from a material having good mechanical integrity and wear-resistance. There is also a need for new methods to fabricate night guards. As discussed above, conventional methods involve multiple dental office visits. In some instances waxing, investment, and boiling-out techniques are used and this requires skilled dental technicians to practice. Also, night guards made from two different materials with multi-layered structures are costly and time-consuming to fabricate. Ideally, the dentist should be able to design and fabricate the night guard "chair-side" and mount the night guard in a patient's mouth in a single office visit.

The present invention provides for a method for making dental guards (dental arches) as defined in claim 12.

### SUMMARY OF THE INVENTION

The present invention provides a kit for making dental guards that can be used by patients to protect their teeth against the effects of grinding and clenching. The invention also provides methods for making such dental guards. The dentist uses a light-curable polymerizable arch material to fabricate the dental guard in accordance with this invention. The dental guard has a U-shaped structure with opposing upper and lower planar surfaces and inner (lingual) and outer (buccal) surfaces. The dental guard is made from an arch material that is capable of being molded to conform to a shape of a dental arch (upper or lower) of a patient. The arch material is shape-stable at room temperature and 37°C (normal temperature inside of the mouth) and cured by light irradiation to retain its molded shape. The polymerizable material contains a polymerizable acrylic compound, and polymerization system capable of being activated by light, and preferably color-changing indicator, particulate filler.

In one method for making the dental guard, the dental arch material can be placed in the patient's mouth and molded over the upper or lower dental arch. A curing light can be used to partially cure the arch material inside of the mouth. Then, the partially-cured dental arch can be removed from the mouth and irradiated with light in a second light-curing step so that it fully cures. Blue visible light having a wavelength in the range of about 400 to about 500 nm can be used in the first and second light-curing steps. The invention also provides kits for making the dental guard. These kits include the U-shaped dental guard contained in a flexible tray that can be made from silicone. A U-shaped backing film, for example, Parafilm (hydrocarbon wax film), is placed over the dental guard and a release liner is placed over the backing film. The kit can be vacuum-sealed in a light-protective package for shipping and handling.

### BRIEF DESCRIPTION OF THE DRAWINGS

The novel features that are characteristic of the present invention are set-forth in the appended claims. However, the preferred embodiments of the invention, together with further objects and attendant advantages, are best understood by reference to the following detailed description in connection with the accompanying drawings in which:
FIG. 1 is a perspective view of one embodiment of a tray comprised in the kit of the present invention, for holding a dental arch ;
FIG. 1A is a top view of the tray shown in FIG. 1 used for holding the dental arch;
FIG. 1B is a front perspective view of the tray shown in FIG. 1 used for holding the dental arch;
FIG. 1C is a side perspective view of the tray shown in FIG. 1 used for holding the dental arch;
FIG. 2 is a perspective view of a dental arch with film backing adhered to the lower surface of the arch;
FIG. 2A is a perspective view of a dental arch with film backing adhered to the upper surface of the arch;
FIG. 3 is a perspective view of the dental arch by itself;
FIG. 4 is a top view of the dental arch with film backing and release liner positioned in the tray;
FIG. 5 is a front perspective view showing the dental arch material being applied by finger-pressure over the upper dental arch of a patient;
FIG. 5A is a front perspective view showing the dental arch material being light-cured to form a dental guard; and
FIG. 5B is a front perspective view showing the finished dental guard fitted in position over the upper dental arch of a patient.

### DETAILED DESCRIPTION OF THE PREFERRED EMBODIMENTS

The present invention relates to methods and kits for making dental guards. In accordance with this invention, a dentist uses a light-curable polymerizable arch material to fabricate the guard. The dentist can fabricate and mount the dental guard in a patient's mouth during a single office visit. The dental guard is described primarily herein as being a night guard intended for a patient to wear during the night time. However, it should be understood that the dental guard of this invention can be worn at any time of the day. The dental guard obtainable according to this invention is particularly effective in protecting teeth against the effects of bruxism.

It should also be understood that the light-curable polymerizable arch material can be used to fabricate other dental prosthesis and appliances including, for example, implant stents, bite registrations, crown and bridges, baseplates, splints, denture liners, custom trays, artificial teeth, repairs for natural teeth, veneers, denture repairs, denture relines, retainers, orthodontic components, provisional dental devices, inlays, onlays, orthodontic appliances, temporary dentures, temporary partial dentures, maxillofacial prostheses, obturators, and occular prostheses, and the like.

### Material

### Polymerizable Acrylic Compounds

Polymerizable acrylic compounds that can be used in the photopolymerizable material used to make the night guard of this invention, include, but are not limited to, mono-, di- or polyacrylates and methacrylates such as methyl acrylate, methyl methacrylate, ethyl acrylate, isopropyl methacrylate, n-hexyl acrylate, stearyl acrylate, allyl acrylate, stearyl methacrylate, the reaction product of octadecyl isocyanate and 2-hydroxyethyl methacrylate, the reaction product of octadecyl isocyanate and caprolactone 2-(methacryloyloxy)ethyl ester, the reaction product of octadecyl isocyanate and 2-hydroxyethyl acrylate, the reaction product of octadecyl isocyanate and hydroxypropyl (meth)acrylate, the reaction product of octadecyl isocyanate and 2-hydroxypropyl 2-(methacryloyloxy)-ethyl phthalate, the reaction product of octadecyl isocyanate and 2-hydroxy-3-phenoxypropyl acrylate, the reaction product of octadecyl isocyanate and glycerol dimethacrylate, the reaction product of octadecyl isocyanate and pentaerythritol triacrylate, etc., the reaction product of cyclohexyl isocyanate and 2-hydroxyethyl (meth)acrylate, the reaction product of benzyl isocyanate and 2-hydroxyethyl (meth)acrylate, etc., diethyleneglycol diacrylate, triethyleneglycol dimethacrylate, tetraethylene glycol di(meth)acrylate, 1,3-propanediol diacrylate, 1,3-propanediol dimethacrylate, trimethylolpropane tri(meth)acrylate, 1,2,4-butanetriol trimethacrylate, 1,4-cyclohexanediol diacrylate, 1,4-cyclohexanediol dimethacrylate, 1,6-hexanediol di(meth)acrylate, pentaerythritol triacrylate, pentaerythritol tetraacrylate, pentaerythritol tetramethacrylate, sorbitol hexacrylate, 2,2-bis[4-(2-hydroxy-3-acryloyloxypropoxy)phenyl]propane; 2,2-bis[4-(2-hydroxy-3-methacryloyloxypropoxy)phenyl]propane (Bis-GMA); 2,2-bis[4-(acryloyloxy-ethoxy)phenyl]propane; 2,2-bis[4-(methacryloyloxy-ethoxy)phenyl]propane (or ethoxylated bisphenol A-dimethacrylate) (EBPADMA).

Urethane acrylates and methacrylates can be used. These include urethane di(meth)acrylate (UDMA), diurethane dimethacrylate (DUDMA), polyurethane dimethacrylate (PUDMA); 4,13-dioxo-3,14 dioxa-5,12-diazahexadecane-1,16-diol diacrylate; 4,13-dioxo-3,14 dioxa-5,12-diazahexadecane-1,16-diol dimethacrylate; aliphatic polyester urethane niethacrylate; the reaction product of trimethyl 1,6-diisocyanatohexane and bisphenol A propoxylate and 2-hydroxyethyl methacrylate (TBDMA;); CN962 (sold by Sartomer Company, Exton, PA), CN964 (sold by Sartomer Company, Exton, PA), SR480 (sold by Sartomer Company, Exton, PA), CD540 (sold by Sartomer Company, Exton, PA), (the reaction product of 1,6 diisocyanatohexane and 2-hydroxyethyl methacrylate modified with water (HDIDMA); the reaction product of 1,6 diisocyanatohexane and 2-hydroxyethyl acrylate modified with water (HDIDA); alkoxylated pentacrythritol tetraacrylate; polycarbonate dimethacrylate (PCDMA); the bis-acrylates and bis-methacrylates of polyethylene glycols; the bis-acrylates and bis-methacrylates of ethoxylated/propoxylated/or alkoxylated bisphenol A; and copolymerizable mixtures of acrylated monomers and acrylated oligomers.

In addition to the foregoing polymerizable acrylic compounds, the composition may contain acidic monomers such as dipentaerythritol pentacrylate phosphoric acid ester (PENTA); bis[2-(methacryloxyloxy)-ethyl]phosphate; and vinyl compounds such as styrene, diallyl phthalate, divinyl succinate, divinyl adipate and divinylphthalate.

### Polymerization System

A polymerization system, which initiates polymerization (hardening) by a light-curable reaction, is used in the material of this invention. In one embodiment, a photoactive agent such as, for example, benzophenone, benzoin and their derivatives, or alpha-diketones and their derivatives is added to the composition in order to make it light-curable. A preferred photopolymerization initiator is camphorquinone (CQ). As discussed further below, photopolymerization of the composition occurs following a two-step process. Polymerization is initiated by irradiating the composition with blue, visible light preferably having a wavelength in the range of about 380 to about 500 nm. A standard dental blue light-curing unit can be used to irradiate the composition. Photoinitiators selected from the class of acylphosphine oxides can also be used. These compounds include, for example, monoacyl phosphine oxide derivatives, bisacyl phosphine oxide derivatives, and triacyl phosphine oxide derivatives. For example, 2, 4,6-trimethylbenzoyl-diphenyl-phosphine oxide (Lucirin-TPO) can be used as the photopolymerization initiator. In one embodiment, a material referred to as "ALF" comprising camphorquinone (CQ); butylated hydroxytoluene (BHT); N,N-dimethylaminoneopentyl acrylate, γ-methacryloxypropyltrimethoxysilane and methacrylic acid can be used in the composition.

In addition to the photoactive agents, the composition may include a polymerization inhibitor such as, for example, butylated hydroxytoluene (BHT); hydroquinone; hydroquinone monomethyl ether; benzoquinone; chloranil; phenol; butyl hydroxyanaline (BHT); tertiary butyl hydroquinone (TBHQ); tocopherol (Vitamin E); and the like. Preferably, butylated hydroxytoluene (BHT) is used as the polymerization inhibitor. The polymerization inhibitors act as scavengers to trap free radicals in the composition and extend the composition's shelf life.

### Color Indicators

Color-changing agents can be added to the composition of this invention. These cure indicators can be added to monitor the partial curing of the arch material. Once the dental practitioner discerns the color change of the arch material, he or she knows the dental guard is partially-cured and has sufficient dimensional-stability to be safely removed from the mouth without distortion.

In addition, irradiation and polymerization indicators and photobleachable dyes can be added into the composition of this invention. Examples include those disclosed in US Patent Publication 2006/0280649 (Grundler), US Patent Publication 2006/0194895 (Loveridge, et al), US Patent 6,960,079 (Brennan, et al), US Patent 6,890,399 (Wojciak), and US Patent 6,528,555 (Nikutowski, et al).. Preferred materials include, for example, photobleachable dyes, fluorescent materials, photochromic materials, and phosphorescent materials. Preferred dyes include, for example, Rose Bengal, Methylene Blue, Methylene Violet, Eosin Yellow; Eosin B, Eosin Y, Eosin G, Ethyl Eosin, Toluidine Blue, Erythosin B, Cresyl Violet, 4',6-diamidino-2-phenylindole (DAPI, blue fluorescence), Fluorescein, 4',5'-Dibromofluorescein, etc. and their combinations. 4-[(octyloxy)phenyl]phenyl iodonium hexafluoroantimonate (OPPI), diphenyliodonium hexafluorophosphate (HPIHFP), etc. may also be added to the composition.

### Fillers

Conventional filler materials may be added to the composition. These include inorganic and organic fillers. Such conventional materials include, but are not limited to, silica, titanium dioxide, iron oxides, silicon nitrides, glasses such as calcium, lead, lithium, cerium, tin, zirconium, strontium, barium, and aluminum-based glasses, borosilicate glasses, strontium borosilicate, barium silicate, lithium silicate, lithium alumina silicate, kaolin, quartz, and talc. The average particle size of the inorganic filler particles is normally in the range of about 0.005 to about 10 µm, more preferably in the range of about 0.01 to about 5 µm, and most preferably in the range of about 0.01 to about 1 µm.

Organic particles such as poly(methyl methacrylate), poly(methyl/ethyl methacrylate), crosslinked poly(meth)acrylates, polyurethanes, polyethylene, polypropylene, polycarbonates and polyepoxides , and the like also can be used as fillers.

In addition or as an alternative to such conventional filler materials, the above-described polymerizable acrylic compounds can be cured and ground to form particulate powder. These hardened powder granules can be added as a filler material to the dental composition. The polymerizable dental compositions may include from 0 to about 90 percent by weight filler material. In a preferred embodiment, the compositions include from about 2 to about 75 percent by weight filler and more preferably from about 5 to about 50 percent by weight. In one embodiment, the composition may contain 10 wt.% of polymerized acrylic compound (as described above) in the form of powder particulate and 90 wt% polymerizable acrylic compounds in the form of resin. As described in the examples below, a composition containing 10 wt.% powder particulate (polymerized acrylic compound) is less sticky and tacky than compositions that do not contain 10 wt.% powder particulate. The average particle size of the powder particulate granules (prepared from polymerized acrylic compounds) is normally less than 1,000 µm, more preferably less than 200 µm, and most preferably less than 100 µm .

In one preferred embodiment, the composition comprises about 5 to about 50 wt.% TBDMA; about 5 to about 20 wt.% CAP-SMA (4,11-dioxo-3,10-dioxa-12-azatriacontane-1-ol methacrylate); about 5 to about 20 wt.% G4256 (aliphatic polyester urethane methacrylate, available from Rahn USA Corp, Aurora, IL); about 1 to about 10 wt.% SR 348 (ethoxylated₂ bisphenol A dimethacrylate, available from Sartomer Company, Exton, PA); about 1 to about 5 wt.% ODA (Octadecyl acrylate); about 0.1 to about 5 wt.% Lucirin-TPO (2,4,6-trimethylbenzoyldiphenylphosphine oxide available from BASF.) and 0.1 to 4% ALF.

In another preferred embodiment, the composition comprises about 10 to about 30 wt.% TBDMA; about 10 to about 30 wt.% CAP-SMA (4,11-dioxo-3,10-dioxa-12-azatriacontane-1-ol methacrylate); about 20 to about 60 wt.% G4256 (aliphatic polyester urethane methacrylate, available from Rahn USA Corp, Aurora, IL); about 5 to about 20 wt.% CN962 (urethane acrylate, available from Sartomer Company, Exton, PA); about 0.1 to about 5 wt.% Lucirin-TPO (2,4,6-trimethylbenzoyldiphenylphosphine oxide available from BASF.) and 0.1 to 4% ALF.

This composition provides a soft, resilient polymerized material that forms a soft layer in a dental guard.

In yet another preferred embodiment, the composition comprises about 5 to about 50 wt.% TBDMA; about 5 to about 20 wt.% CAP-SMA (4,11-dioxo-3,10-dioxa-12-azatriacontane-1-ol methacrylate); about 5 to about 20 wt.% G4256 (aliphatic polyester urethane methacrylate, available from Rahn USA Corp, Aurora, IL); about 1 to about 10 wt.% SR 348 (ethoxylated₂ bisphenol A dimethacrylate, available from Sartomer Company, Exton, PA); about 1 to about 5 wt.% ODA (Octadecyl acrylate); about 0 to 0.01 wt.% Methylene Blue, about 0 to 0.01 wt% Rose Bengal, about 0 to 0.5 wt% OPPI, about 0 to 0.5 wt% DPIHFP, about 0 to 5 wt.% Lucirin-TPO (2,4,6- trimethylbenzoyldiphenylphosphine oxide available from BASF.) and 0.1 to 4 wt% ALF. This composition will change colors visibly upon being partially-cured.

TBDMA is added to the composition in the form of semi-solid high molecular weight oligomers. Cap-SMA is a semi-crystalline monomer. The addition of TBDMA and CAP-SMA provide the composition with good handling properties. Lucirin-TPO and ALF are photoinitiators that initiate the polymerization of the monomers and oligomers and provides a relatively short cure time. Organic filler materials are added to improve the handling properties of the composition. In addition, the use of organic filler materials also reduces polymerization shrinkage.

The material used in the method of this invention has some wax-like properties so it can be molded and shaped without additional heating. The geometric shape of the molded material is maintained prior to being cured. That is, the material is dimensionally-stable at 37°C (normal temperature inside of the mouth) and room temperature while it is in its uncured state. "Dimensional-stability" or "shape-stable" as used herein refers to material which maintains its shape as determined by testing methods according to ADA (American Dental Association) consistency test specification 19, Paragraph 4.3.4 (23°C), JAVA Vol. 94, April, 1977, pages 734-737. The material comprises semi-crystalline components that are partially recrystallizable and help the material to solidify. The material forms a surface layer having relatively low stickiness upon being crystallized. When polymerized, the crystallized phase melts effectively resulting in volume expansion, which offsets polymerization shrinkage somewhat. The resulting material has low shrinkage and stress.

### Methods

Following one embodiment of the method of this invention, a dental practitioner applies the wax-like polymerizable arch material over the upper or lower dental arch (teeth) to form the dental guard. The dental arch material is described primarily herein as being mounted over the upper dental arch. However, it should be understood that the arch material can be mounted and molded over the upper or lower dental arches depending upon the needs of the patient. The patient is instructed to bite down on the wax-like material.

When the patient bites down, he/she forms a bite impression in the dental arch material. This bite impression, while in an uncured condition, is shape-stable. However, if sufficient pressure is applied to the bite impression, it can be reshaped. Preferably, the patient bites down on a backing film that overlays the occlusal surface of the wax-like arch material as described further below. As the patient bites down, the margins, contacts, and bite occlusion of the arch material are checked by the practitioner. The patient bites down by gently tapping and slowly making disocclusion movements to record occlusal relief on the arch material. Checking the arch material in the manner provides optimum fit so less adjustment will be needed later.

Next, the shaped arch material structure is partially-cured in the mouth to form the dental guard, preferably a night guard. Standard Light-Emitting Diode (LED), halogen, and plasma arc (PAC) handheld dental curing lights may be used to partially cure the material. Suitable dental curing lights include, for example, those sold under the brand names: SmartLite® iQ2™ and PS™ (Dentsply); Elipar® (3M Espe); L.E. Demetron II™ (Kerr); and Bluphase™ (Ivoclar Vivadent); QHL75® Lite (Dentsply); Spectrum® 800 (Dentsply); and Sapphire (DenMat).

The curing light partially cures the material by activating the photopolymerization system in the material. Particularly, the camphorquinone (CQ) compounds have a light absorbency range of between about 420 to about 500 nm and generate free radicals for polymerization when irradiated with light having a wavelength in this range. The composition thus begins to harden and forms a dental guard structure.

It can be difficult to distinguish between partially-cured materials versus non-cured materials. The dental practitioner may have a difficult time detecting when the night guard material has been partially-cured. To address this problem, a cure-indicator may be added to the composition. Suitable cure-indicators that can be used in the composition are described above. The cure-indicator has a first color that imparts substantial color to the composition. When the composition is partially-cured, the cure-indicator transforms into a second color that is different from the initial color. This second color is imparted to the composition. An unaided human eye can instantaneously see the difference between second color and first color of the composition once it has been partially-cured. The second color is distinguishable so the dental practitioner can immediately discern the partial curing of the composition.

Next, the dentist removes the partially-cured dental guard structure from the mouth. The dentist may use an explorer, probe, or other instrument to detach the dental guard from the teeth. Under ordinary circumstances, the dentist would have difficulty removing a traditional dental guard with high rigidity and modulus. However, because the material is only partially-cured at this point and the wax-like material of this invention is flexible with relatively low modulus, the dentist is able to remove the dental guard easily. Moreover, the dental guard is flexible, enough to accommodate undercuts on the teeth. The partially-cured material does not have full hardness and does not strongly adhere to the tooth surfaces. However, at the same time, the partially-cured material does have sufficient integrity and stability such that the shape of the dental guard is maintained. There is no deformation of the partially-cured dental guard as it is removed.

In addition, because the dental guard is made from a flexible material, it is easier to remove from the patient's mouth. The dental guard is soft and pliable so that it can be handled more effectively. At the same time, the dental guard has good mechanical integrity so that it does not fracture or break. Preferably, the dental guard is made of a material comprising an aliphatic polyester urethane methacrylate which imparts these advantageous properties.

After removing the dental guard from the mouth, the dentist may inject a rigid material (for example, die silicone) inside of the dental guard to form a cast so as to minimize the potential distortion. Then, the dental guard is fully cured in a second curing step. A standard light-curing oven may be used to fully cure the dental guard. Suitable light-curing ovens include, for example, the Eclipse® processing unit, Enterra® visible light-curing (VLC) unit, and Triad® 2000 VLC unit available from Dentsply.

The fully cured dental guards are resilient and flexible as well as mechanically strong. In general, the fully cured dental guards have a flexural strength of less than 10,000 psi at 37°C and a flexural modulus of less than 2.068 GPa (300,000 psi) at 37°C. In one preferred embodiment, the dental guards have a flexural strength of about 7MPa and a flexural modulus of 200 MPa at 37°C. A material with low flexural modulus means that a relatively low amount of force is required to deform or deflect the material. Dental guards having low flexural modulus are advantageous, because they are flexible and can be placed in a mouth and removed therefrom easily. Because of the dental guard's soft and pliable nature, it fits more easily over the contours of the tooth. The dental guard is more comfortable to wear.

After completing the second light-curing step, the dental guard can be finished using a standard dental bur or other suitable instrument to remove excess materials and smooth any rough spots. The finished and polished dental guard is ready to be positioned inside of the mouth of a patient.

In an alternative method, the dental guard is not partially-cured inside of the patient's mouth. Instead, the dental practitioner warms and places the wax-like polymerizable arch material over the upper or lower dental arch of the patient. The patient bites down upon the arch material to form an impression therein. The material is molded so that it conforms to the patient's dentition. The material cools and forms a stable, uncured dental guard structure inside of the patient's mouth. The dental guard structure is then removed from the mouth. The dental guard structure is fully cured by exposing it to light radiation outside of the mouth using standard dental curing lights or ovens as described above. This method can be advantageous, because it allows the dentist the chance to work extraorally to prepare the dental guard structure.

In another embodiment, the night guard is a laminated structure comprising a first polymerized layer and an overlying second polymerized layer. The first layer forms the exterior of the night guard - it is a relatively hard layer. The second layer, which is laminated to the first layer, forms the interior surface of the night guard - it is a relatively soft layer and will make contact with the tooth surface. The interior layer provides a soft cushion for the tooth surfaces as the patient bites down into the night guard, while the exterior layer provides a harder protective tooth guard. The above-described polymerizable acrylic compounds, polymerization systems, and fillers can be used to prepare the first and second layers of the night guard, provided that, the composition of the first layer is different than the second layer.

Following the methods of this invention, a dental guard can be made at the side of a dental chair in a dental office. The dental practitioner can design and fabricate the dental guard "chairside" and mount the guard in a patient's mouth in a single office visit. The finished dental guard has good durability, wear-resistance, and flexibility.

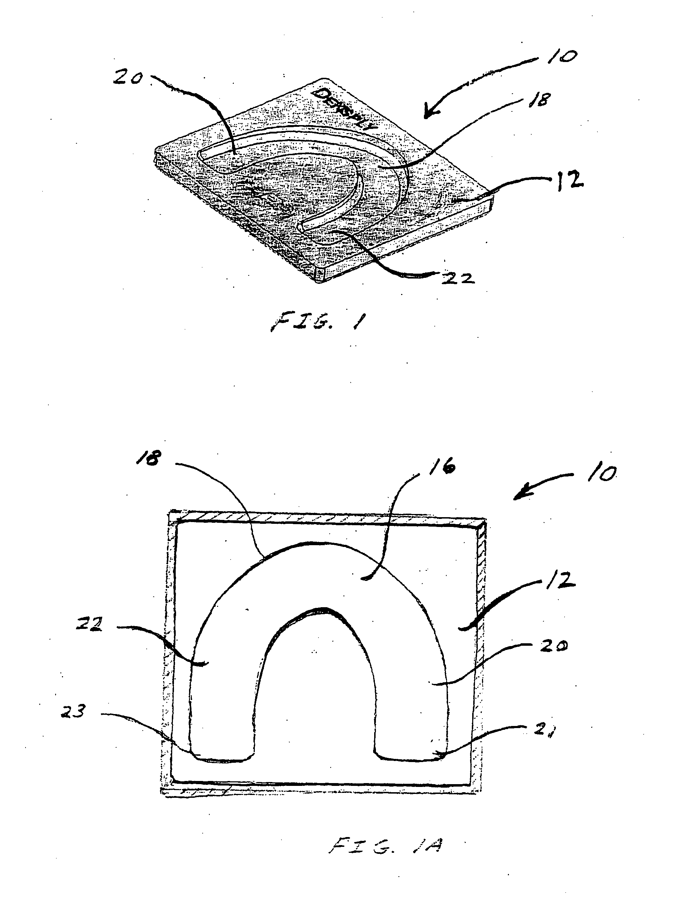

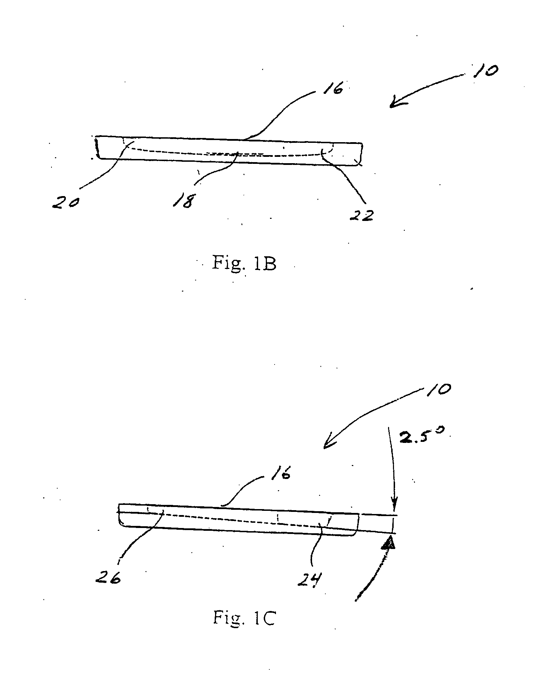

This invention also provides kits for fabricating the dental guard. The kits can be provided to the dentist so that he/she can make the dental guard chairside for a patient. Referring to FIGS. 1 to 1C the kits contain a flexible tray (10) having upper and lower planar surfaces (12, 14). The upper surface (12) of the tray (10) includes a U-shaped recessed portion or well (16) for holding the U-shaped dental guard (not shown) described further below. As shown in FIG. 1B, the central segment (18) of the U-shaped recessed well (16) is relatively deep. The recessed well (16) is sloped up slightly towards the extending leg segments (20, 22). The tips (21,23) of the leg segments (20, 22) are slightly rounded and have small radii. As shown in FIG. 1C, the anterior region (24) of the U-shaped recessed well (16) is relatively deep. Moving towards the posterior region (26), the depth tapers off so the posterior region (26) of the recessed well (26) is more shallow than the anterior region (24).

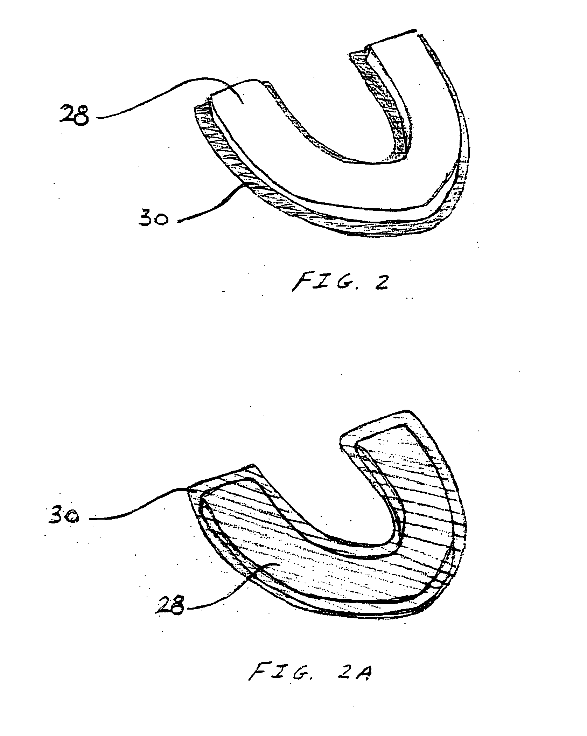

Referring to FIGS. 2 and 2A, the U-shaped dental arch (28), which is retained in the tray (10), is shown. The dental arch (28) is used to fabricate the dental guard of this invention. The dental arch (28) has a U-shaped structure designed to fit over the upper or lower dental arch of the patient. A U-shaped film backing (30) is placed over the U-shaped dental arch (28).

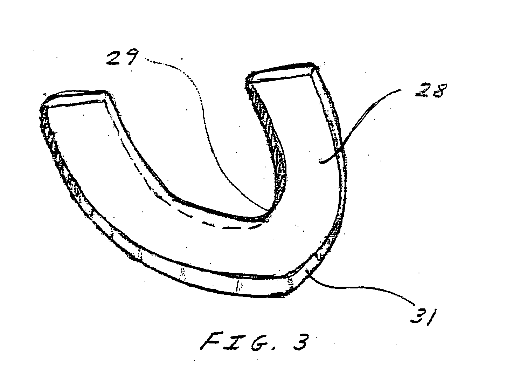

In FIG. 2, the film backing (30) is shown positioned over the lower surface of the dental arch (28), and in FIG. 2A, the film backing (30) is placed over the upper surface of the dental arch (28). The film backing (30) allows for easy handling of the dental arch (28) and minimizes stickiness. The dental arch (28) can become sticky when it is handled with extensive hand manipulation, especially at elevated temperatures inside of the patient's mouth. The use of a film backing (30) helps minimize stickiness. In addition, the film backing (30) creates a barrier that cases the bite-in of opposing dentition when the patient bites down. Furthermore, when a handheld dental curing light is used to cure the dental guard during initial partial polymerization as described above, the tip of the curing light often makes contact with the dental guard. The film backing (30), prevents the dental guard from becoming contaminated by the curing light tip. Conversely, the film backing (30) prevents the curing light tip from becoming contaminated by dental guard resin. In FIG. 3, the shape-stable, wedge-shaped dental arch (28), by itself, is shown. The dental arch (28) comprises a U-shaped base material having upper and lower planar surfaces and inner (lingual) and outer (buccal) surfaces (29, 31). The anterior region of the wedge-shaped dental arch (28) is relatively thick. Moving towards the posterior region, the thickness tapers off so the posterior region of the wedge-shaped dental arch (28) is thinner than the anterior region. The sloped angle of the wedge-shaped dental arch (28) can vary and is normally in the range of about two to three degrees. The dental arch (28) can be fabricated in a variety of sizes (for example, small, medium, and large) to fit different patients. The practitioner selects the approximately-sized dental arch to fit the patient and can trim it as needed.

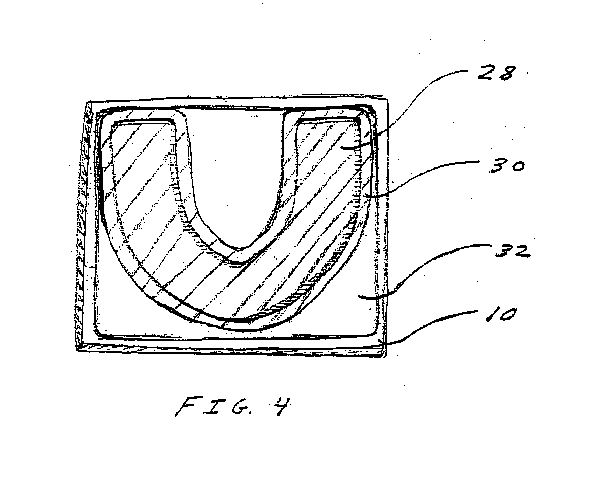

Referring to FIG. 4, a release liner or sheet material (32) can be placed over the tray (10) before it is packaged. The release liner (32) helps to maintain the integrity of the packed dental guard during transportation. The release liner (32) protects the dental guard and film backing (30) from sticking to the inside of the package. Additionally, the release liner (32) protects the dental arch (28) from contamination and debris. The U-shaped film backing (30) can be made from elastic thermoplastics such as polyurethane, polyethylene, polypropylene, polyvinylidene, cellulose acetate, polyether, polyester, polyvinyl chloride and their copolymers, etc. Preferably, the U-shaped backing (30) is made of Parafilm M (hydrocarbon wax film a product of the Pechiney Plastic Packaging Company). The tray (10) containing the dental arch (28) can be packaged and vacuum sealed in a light-protective envelope or other container (not shown). Vacuum sealing procedures are used to prevent contamination and help prevent the packaged dental guard from damage during shipping and handling.

Similarly, this invention provides a laminated wedge-shaped dental arch and kit, which can be used to fabricate a night guard with a soft layer for added comfort, easy insertion and removal to the patient and a hard occlusal surface layer with good wear resistance for long-term durability. It also provides dentist and dental professional with a simple and fast technique to fabricate a hard/soft night guard conveniently.

In practice, the dentist removes the tray containing the dental arch from the sealed light-protective package. The dentist holds the tray in one hand. He or she can then peel-off the release liner with their other hand. Once the release liner has been peeled away, the clinician can easily remove the dental arch with the protective U-shaped backing film from the tray. The release liner should be set aside, because it can be used in the curing process as described further below. Only the release liner should be removed - the U-shaped film backing which covers the upper surface of the U-shaped dental arch should remain in place. The clinician can grasp the dental arch by pinching it between his/her thumb and finger and pulling it out of the recessed well in the tray. The tray is preferably made of a silicone, rubber, or other elastomeric material. This material has several advantageous properties; particularly, it provides the tray with sufficient mechanical integrity and rigidity to hold the U-shaped dental arch. At the same time, the material is resilient so it will bend and flex slightly. The practitioner thus can pinch the tray with his/her thumb and finger and squeeze it. This causes the U-shaped dental arch to "pop out" slightly from the U-shaped well of the tray. The practitioner can then just simply pull the U-shaped dental arch out of the tray.

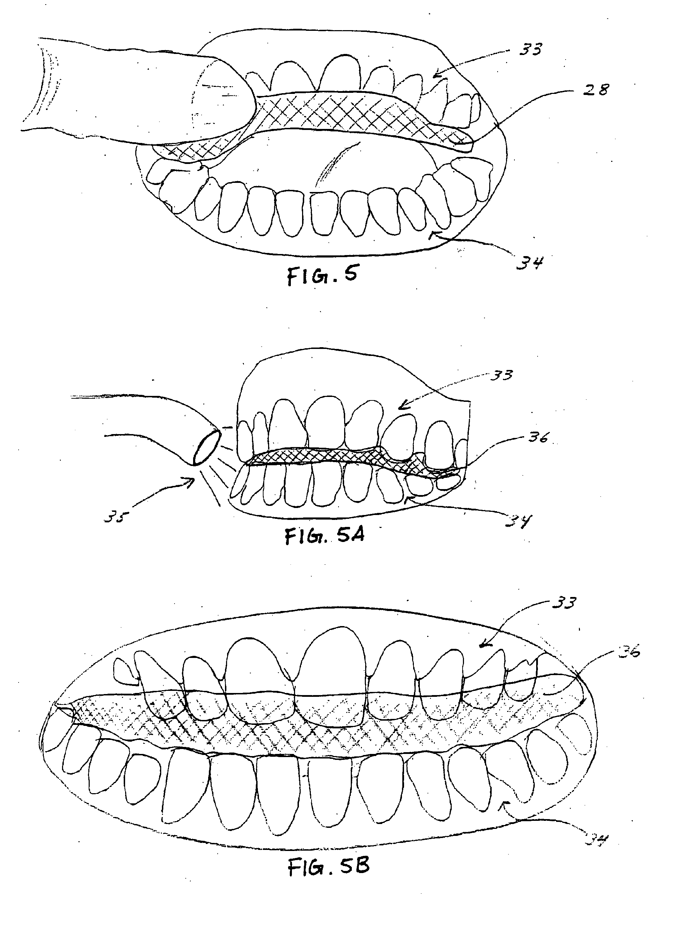

Using a pair of scissors or other sharp instrument, the practitioner may trim the dental arch so that it will better fit the dentition of the patient. The tooth surfaces should be wet. Then, the practitioner inserts the dental arch, which includes one surface still covered by the film backing and a second exposed surface, into the mouth. Referring to FIGS. 5-5B, the exposed resinous surface of the dental arch (28) is pressed against the wet tooth surfaces, while the film-covered surface of the arch (28) can be molded easily with minimal stickiness using finger-pressure. The film backing (30) prevents opposing teeth from sticking to the dental arch (28) s the patient bites down. As shown in FIG. 5, for example, if the arch material (28) is mounted over the upper dentition, then the upper surface of the arch is exposed and pressed against the upper tooth surfaces. And, the lower surface of the arch material (28) remains covered by the film backing - this prevents the lower .teeth from sticking to the guard.

With finger-pressure, the practitioner adapts the arch material (28) onto the mid-facial tooth surfaces while covering most of the lingual surfaces. The material is adapted into the tooth embrasures while avoiding deep undercuts. The patient bites down lightly on the guard several times and goes through lateral jaw excursions. After the occlusal contacts have been registered in the film backing, the patient bites down lightly and holds that position while the practitioner directs curing light radiation (35) against the facial tooth surfaces (FIG. 5A). The practitioner may wish to use the light guide tip of the curing light (35) to further press and contour the guard (36) against the tooth surfaces. After irradiating the facial tooth surfaces with curing light for a sufficient time period, the patient opens his/her mouth so that the curing light can be directed against the lingual surfaces. Finally, the curing light is directed against the occlusal surfaces. The amount of time needed to sufficiently partially cure the guard will vary depending upon the type of curing light used, patient condition, and other factors. In general, the dental guard can be sufficiently partially-cured after about three minutes of intra-oral curing using a quartz halogen curing light and after about four and one-half minutes using a LED curing light. The partially-cured dental guard with its film backing is then removed from the mouth. It is now ready to be fully cured and finished.

The resinous dental guard contains tooth impressions as a result of the patient biting down on the guard. It is recommended that these tooth spaces be filled with a die silicone material before the second curing step. The tooth spaces can be filled with Regisil® rigid die silicone (Dentsply). The die silicone filled guard is inverted and placed face down on the square-shaped release liner used in the original packaging as described above. The die silicone normally sets after about two minutes. Now, the dental guard is ready to be fully cured. It is placed in a visible light-curing unit and irradiated with curing light so that it fully cures. The amount of time needed to fully cure the guard will vary depending upon the type of light-curing unit used. For example, the following curing units from Dentsply can be used: Eclipse® processing unit, Enterra® visible light-curing (VLC) unit, and Triad® 2000 VLC unit. In general, the curing cycle is normally in the range of about 5 to about 20 minutes. The finished dental guard (36) can be mounted over the upper dental arch in the mouth of a patient as shown in FIG. 5B.

The present invention is further illustrated by the following Examples and Test Methods, but these should not be construed as limiting the scope of the invention.

### Test Methods

### Flexural strength and flexural modulus

The flexural strength and flexural modulus properties of the materials were measured according to the test methods in ASTM D790 (1997). A material with low flexural modulus means that a relatively low amount of force is required to deform or deflect that material.

### Un-notched impact strength

The un-notched impact strength of the materials was measured according to the test methods in ASTM D4812 (1993).

### Tackiness

The tackiness of the polymerizable material's was measured according to the test methods in ASTM D3121-06 (modified). The test methods were modified by: 1) reducing the ball diameter to 6.35 mm (1/4 inch) from 11.11 mm (7/16 inch), and 2) reducing the length of ramp used (to accelerate the ball) to 25.4 mm (1 inch) from 165.1 mm (6.5 inches) . Following this procedure, the stickiness of the material is based upon the distance that the ball travels along the ramp. The longer distance the ball travels, the less sticky and tacky the material is.

### EXAMPLES

### Preparation 1. Preparation of TBDMA Oligomer

A reactor was charged with 1176 grams of trimethyl-1,6-diisocyanatohexane (5.59 mol) and 1064 grams of bisphenol A propoxylate (3.09 mol) under dry nitrogen flow and heated to about 65°C. under positive nitrogen pressure. To this reaction mixture, 10 drops of catalyst dibutyltin dilaurate were added. The temperature of the reaction mixture was maintained between 65 °C and 140 °C for about 70 minutes and followed by additional 10 drops of catalyst dibutyltin dilaurate. A viscous paste-like isocyanate end-capped intermediate product was formed and stirred for 100 minutes. To this intermediate product, 662 grams (5.09 mol) of 2-hydroxyethyl methacrylate and 7.0 grams of BHT as an inhibitor were added over a period of 70 minutes while the reaction temperature was maintained between 68°C and 90°C. After about five hours stirring under 70°C, the heat was turned off, and TBDMA oligomer was collected from the reactor as semi-translucent flexible solid and stored in a dry atmosphere.

### Preparation 2. Preparation of Monomer

A reaction flask was charged with 151.25 grams of octadecyl isocyanate and heated to about 70°C under a positive nitrogen pressure. To this reactor were added 125.3 grams of caprolactone 2-(methacryloyloxy)ethyl ester, 0.12 gram of catalyst dibutyltin dilaurate and 0.58 grams of butylated hydroxy toluene (BHT). The addition was slow and under dry nitrogen flow over a period of two hours. The temperature of the reaction mixture was maintained between 70°C and 85°C for another 2.5 hours, the reaction product was discharged as clear liquid into plastic containers and cooled to form a semi-opaque solid and stored in a dry atmosphere.

### Example 1. Polymerizable Material

A light-curable polymerizable material was prepared by stirring and degassing at 85°C a liquid of 69.0 grams of TBDMA oligomer prepared by following the procedure of Preparation 1, 11.0 grams of monomer prepared by following the procedure of Preparation 2, 12.0 grams of Genomer 4256 (available from Rahn USA), 6.0 grams of SR348 (sold by Sartomer), 1.0 grams of octadecyl acrylate (ODA), 0.5 gram of 2,4,6- trimethylbenzoyldiphenylphosphine oxide (Lucirin TPO made by BASF) and 0.5 gram of visible light initiating solution. Visible light initiating solution contains 13.3% camphorquinone (CQ), 23.0% methacrylic acid (MAA), 1.3% butylated hydroxytoluene (BHT), 46% N, N-dimethylaminoethylneopentyl acrylate, 16.3% γ-methacryloxypropyltrimethoxysilane and 66.7% 1,6-hexanediol dimethacrylate (HDDMA).

### Example 2. Polymerizable Material

A light-curable polymerizable material was prepared by stirring and degassing at 85°C a liquid of 65.5 grams of TBDMA oligomer prepared by following the procedure of Preparation 1, 14.2 grams of monomer prepared by following the procedure of Preparation 2, 11.0 grams of Genomer 4256 (available from Rahn USA), 4.4 grams of SR348 (sold by Sartomer), 2.0 grams of octadecyl acrylate (ODA), 0.7 gram of 2,4,6- trimethylbenzoyldiphenylphosphine oxide (Lucirin TPO made by BASF) and 2.2 gram of visible light initiating solution. Visible light initiating solution contains 13.3% camphorquinone (CQ), 23.0% methacrylic acid (MAA), 1.3% butylated hydroxytoluene (BHT), 46% N, N-dimethylaminoethylneopentyl acrylate, 16.3% γ-methacryloxypropyltrimethoxysilane and 66.7% 1,6-hexanediol dimethacrylate (HDDMA).

### Example 3. Polymerizable Material

A light-curable polymerizable material was prepared by stirring at 85°C a liquid of 94.0 grams of TBDMA oligomer prepared by following the procedure Preparation 1, 3.65 grams of octadecyl acrylate (ODA) and 2 grams of monomer prepared by following the procedure of Preparation 2, 0.25 gram of 2,4,6- trimethylbenzoyldiphenylphosphine oxide, (Lucirin TPO made by BASF) and 1.0 gram of visible light initiating solution. Visible light initiating solution contains 13.3% camphorquinone (CQ), 23.0% methacrylic acid (MAA), 1.3% butylated hydroxytoluene (BHT), 46% N, N-dimethylaminoethylneopentyl acrylate, and 16.3% γ-methacryloxypropyltrimethoxysilane.

### Example 4. Polymerizable Material

A light-curable polymerizable material was prepared by stirring and degassing at 85°C a liquid of 57.2 grams of TBDMA oligomer prepared by following the procedure of Preparation 1, 17.3 grams of monomer prepared by following the procedure of Preparation 2, 17.3 grams of Genomer 4256 (available from Rahn USA), 5.2 grams of SR348 (available from Sartomer), 2.3 grams of octadecyl acrylate (ODA), 0.35 gram of 2,4,6- trimethylbenzoyldiphenylphosphine oxide (Lucirin TPO, available from BASF), and 0.35 gram of visible light initiating solution. Visible light initiating solution contains 13.3% camphorquinone (CQ), 23.0% methacrylic acid (MAA), 1.3% butylated hydroxytoluene (BHT), 46% N, N-dimethylaminoethylneopentyl acrylate, and 16.3% γ-methacryloxypropyltrimethoxysilane. The composition was polymerized with a visible light unit, such as Eclipse® processing unit, and ground to powder having an average particle size less than 100 µm.

### Example 5. Polymerizable Material

A light-curable polymerizable material was prepared by stirring and degassing at 85°C a liquid of 49.6 grams of TBDMA oligomer prepared by following the procedure of Preparation 1, 15.0 grams of monomer formed by following the procedure of Preparation 2, 15.0 grams of Genomer 4256 (sold by Rahn USA), 11.0 grams of polymer powder made in Example 4, 4.5 grams of SR348 (sold by Sartomer), 2.0 grams of octadecyl acrylate (ODA), 2.2 gram of 2,4,6-trimethylbenzoyldiphenylphosphine oxide (Lucirin TPO, made by BASF), and 0.7 gram of visible light initiating solution. Visible light initiating solution contains 13.3% camphorquinone (CQ), 23.0% methacrylic acid (MAA), 1.3% butylated hydroxytoluene (BHT), 46% N, N-dimethylaminoethylneopentyl acrylate, and 16.3% γ-methacryloxypropyltrimethoxysilane.

### Example 6A. Polymerizable Material

A light-curable polymerizable material was prepared by stirring and degassing at 85°C a liquid of 24.0 grams of TBDMA oligomer prepared by following the procedure of Preparation 1, 20.0 grams of monomer prepared by following the procedure of Preparation 2, 44.0 grams of Genomer 4256 (available from Rahn USA), 11.0 grams of CN962 (sold by Sartomer), 0.5 grams of 2,4,6- trimethylbenzoyldiphenylphosphine oxide (Lucirin TPO, available from BASF), and 0.5 gram of visible light initiating solution. Visible light initiating solution contains 13.3% camphorquinone (CQ), 23.0% methacrylic acid (MAA), 1.3% butylated hydroxytoluene (BHT), 46% N, N-dimethylaminoethylneopentyl acrylate, and 16.3% γ-methacryloxypropyltrimethoxysilane. This material will be used to form the soft layer in a laminated dental arch used to make a dental guard by laboratory methods described further below.

### Example 6B. Polymerizable Material

A light-curable polymerizable material was prepared by stirring and degassing at 85°C a liquid of 23.55 grams of TBDMA oligomer prepared by following the procedure of Preparation 1, 19.6 grams of monomer prepared by following the procedure of Preparation 2, 43.15 grams of Genomer 4256 (available from Rahn USA), 10.8 grams of CN962 (sold by Sartomer), 0.7 grams of 2,4,6- trimethylbenzoyldiphenylphosphine oxide (Lucirin TPO, available from BASF), and 2.2 gram of visible light initiating solution. Visible light initiating solution contains 13.3% camphorquinone (CQ), 23.0% methacrylic acid (MAA), 1.3% butylated hydroxytoluene (BHT), 46% N, N-dimethylaminoethylneopentyl acrylate, and 16.3% γ-methacryloxypropyltrimethoxysilane. This material will be used to form the soft layer in a laminated dental arch used to make a dental guard by "chairside" methods described further below.

### Example 7. Preformed Polymerizable Arch Form

About 8 grams of heated (85° C) polymerizable material prepared by following the procedure of Example 5 was formed into an about 2 to 5 mm thick layer in a wedge-shaped silicone arch mold and cooled to form shape-stable wedge-shaped arch. This preformed polymerizable arch can be shaped and cured to form a flexible splint. A thin Parafilm backing and then a plastic releasing film were applied on this uncured wedge-shaped arch in silicone tray and finally vacuum sealed in a light tight plastic package to form a dental arch kit.

### Example 8. Preformed Polymerizable Arch Form

About 8 grams of heated (85° C) polymerizable material prepared by following the procedure of Example 1 was formed into an about 2 to 5 mm thick layer in a wedge-shaped silicone arch mold and cooled to form shape-stable wedge-shaped arch. This preformed polymerizable arch can be shaped and cured to form a flexible splint. A thin Parafilm backing and then a plastic releasing film were applied on this uncured wedge-shaped arch in silicone tray and finally vacuum sealed in a light tight plastic package to form a dental arch kit.

### Example 9A. Preformed Polymerizable Laminated Arch Form with Flexible (Hard) and Resilient (Soft) Layers

About 2 to 3 grams of heated (85° C) polymerizable material prepared by following the procedure of Example 6A was formed into an about 1 to 1.5 mm thick layer in a wedge-shaped silicone arch mold and cooled to form shape-stable arch layer. Then 4 to 5 grams of polymerizable material made by following the procedure of Example 1 was applied on top of above 1 to 1.5 mm thick layer into an about 2 to 5 mm thick laminated layers in a wedge-shaped silicone arch mold and cooled to form shape-stable wedge-shaped arch. This preformed polymerizable laminated arch can be shaped and cured to form a splint having soft and hard layers. A thin Parafilm arch and then a plastic releasing film were applied on this uncured wedge-shaped arch in silicone tray and finally vacuum sealed in a light tight plastic package to form a dental arch kit.

### Example 9B. Preformed Polymerizable Laminated Arch Form with Flexible (Hard) and Resilient (Soft) Layers

About 4 to 5 grams of heated (85° C) polymerizable material prepared by following the procedure of Example 3 was formed into an about 1 to 4 mm thick layer in a wedge-shaped silicone arch mold and cooled to form shape-stable arch layer. Then 2 to 3 grams of polymerizable material made by following the procedure of Example 6A was applied and formed an about 0.5 to 2 mm thick layer on top of above formed layer in a wedge-shaped silicone arch mold and cooled to form shape-stable wedge-shaped laminated arch. This preformed polymerizable laminated arch can be shaped and cured to form a splint having soft and hard layers. A thin Parafilm arch and then a plastic releasing film were applied on this uncured wedge-shaped arch in silicone tray and finally vacuum sealed in a light tight plastic package to form a dental arch kit.

### Example 10. Preformed Polymerizable Laminated Arch Form with Flexible (Hard) and Resilient (Soft) Layers

About 2 to 3 grams of heated (85° C) polymerizable material prepared by following the procedure of Example 6B was formed into an about 1 mm thick layer in a wedge-shaped silicone arch mold and cooled to form shape-stable arch layer. Then 4 to 5 g of polymerizable material made by following the procedure of Example 2 was applied on top of above 1 mm thick layer in a wedge-shaped silicone arch mold and formed a preformed polymerizable laminated arch that can be shaped and cured to form a splint having soft and hard layers. A thin Parafilm arch and then a plastic releasing film were applied on this uncured wedge-shaped arch in silicone tray and finally vacuum sealed in a light tight plastic package to form a dental arch kit.

### Example 11. Fabrication of Flexible Polymeric Night Guard Including Curing Steps

A two part plaster cast model of a patient's teeth was formed and coated with a release agent. A wedge-shaped arch form with an arch Parafilm backing formed by following the procedure of Example 8 was applied over the cast on a two part model, and shaped using finger pressure and trimming to form a polymerizable night guard. The polymerizable composition was further adapted to the two part model as the model was articulated. Air barrier coating (available from Dentsply International under the trademark, Eclipse) might be painted onto the polymerizable night guard and onto the cast as needed. While on the cast the polymerizable night guard was then cured for 6 minutes in a light-curing unit (available from Dentsply International under the trademark: Eclipse®) or 10 minutes in Enterra® (available from Dentsply International). The polymeric night guard formed was clear. Parafilm residue was removed from the cured device as needed and it then might be washed with water to remove all traces of Air Barrier Coating. The night guard was then finished and polished.

### Example 12. Laminated Polymeric Night Guard Having Soft and Hard Layers

A two part plaster cast model of a patient's teeth was formed and coated with a release agent. A wedge-shaped laminated arch form with an arch Parafilm backing formed by following the procedure of Example 9A was applied over the cast on a two part model, and shaped using finger pressure and trimming to form a polymerizable night guard. The polymerizable composition was further adapted to the two part model as the model was articulated. Air barrier coating (available from Dentsply International under the trademark, Eclipse®) might be painted onto the polymerizable night guard and onto the cast as needed. While on the cast the polymerizable night guard was then cured for 6 minutes in a light-curing unit (available from Dentsply International under the trademark, Eclipse®) or 10 minutes in Enterra® (sold by Dentsply International). The polymeric night guard formed was clear. After it was removed from the cast, it was flipped over and air barrier coating (sold by Dentsply International under the trademark: Eclipse) was painted onto the tissue side of night guard and it was then tissue side cured for additional 4 minutes in a light-curing unit (sold by Dentsply International under the trademark: Eclipse® or Enterra®). Parafilm residue was removed from the cured device as needed and it was washed with water to remove all traces of Air Barrier Coating. The night guard was then finished and polished.

### Example 13. Fabrication of Flexible Night Guard Partially-Cured in the Mouth

A wedge-shaped arch form with an arch Parafilm backing prepared by following the procedure of Example 7 was applied over dentition in a patient's mouth. The composition was shaped using finger pressure and trimming to form a night guard. After the night guard was examined and adjusted to fit inside the mouth, it was partially-cured in the mouth for 2 to 3 minutes using a handheld light (sold by Dentsply International under the trademark: QHL75®). The partially-cured night guard was then removed from the mouth. Die Silicone was injected into the cavity of formed night guard. Air barrier coating (sold by Dentsply International under the trademark: Eclipse®) might be painted onto the polymerizable night guard on Die Silicone model and then was cured on the Die Silicone model for 6 minutes in a light-curing unit (sold by Dentsply International under the trademark: Eclipse®) or 10 minutes in Enterra® (sold by Dentsply Interciational). Optional, you may apply air barrier coating first prior to the injection of Die Silicone. The polymeric night guard formed was clear. Parafilm residue was removed from the cured device as needed. Finally, it may be washed with water to remove all traces of Air Barrier Coating. The night guard is then finished and polished.

### Example 14. Fabrication of Laminated Polymeric Night Guard Having Soft and Hard Layers Partially-Cured in the Mouth

A wedge-shaped laminated arch form with an arch Parafilm backing prepared by following the procedure of Example 10 was applied over dentition in a patient's mouth. The composition was shaped using finger pressure and trimming to form a night guard. After the night guard was examined and adjusted to fit inside the mouth, it was partially-cured in the mouth for 2 to 3 minutes using a handheld light (sold by Dentsply International under the trademark: QHL75®). The partially-cured night guard was then removed from the mouth. Handheld.light may be used to further cure the interior surface of night guard arch for 1 minute. Die Silicone was injected into the cavity of formed night guard. Air barrier coating (sold by Dentsply International under the trademark: Eclipse®) might be painted onto the polymerizable night guard on Die Silicone model and then was cured on the Die Silicone model for 6 minutes in a light-curing unit (sold by Dentsply International under the trademark: Eclipse®) or 10 minutes in Enterra® (sold by Dentsply International). Optional, you may apply air barrier coating first prior to the injection of Die Silicone and then may cure the interior surface of night guard for 1 minutes with handheld light. The polymeric night guard formed was clear. After it was removed from the Die Silicone cast, it was flipped over and air barrier coating (sold by Dentsply International under the trademark: Eclipse) was painted onto the tissue side of night guard and it was then tissue side cured for additional 4 minutes in a light-curing unit (sold by Dentsply International under the trademark: Eclipse® or Enterra®). Parafilm residue was removed from the cured night guard as needed. Finally, it may be washed with water to remove all traces of Air Barrier Coating. The night guard is then finished and polished.

### Example 15. Fabrication of Night Guard Cured Without a Die Silicon

A wedge-shaped laminated arch form with an arch Parafilm backing prepared by following the procedure of Example 7 was applied over dentition in a patient's mouth. The composition was shaped using finger pressure and trimming to form a night guard. After the night guard was examined and adjusted to fit inside the mouth, it was partially-cured in the mouth for 2 to 3 minutes using a handheld light (sold by Dentsply International under the trademark: QHL75®). The partially-cured night guard was then removed from the mouth. Air barrier coating (sold by Dentsply International under the trademark: Eclipse®) was painted onto the polymerizable night guard and then was cured for 6 minutes in a light-curing unit (sold by Dentsply International under the trademark: Eclipse®) or 10 minutes in Enterra® (sold by Dentsply International). Parafilm residue was removed from the cured night guard as needed. Finally, it was washed with water to remove all traces of Air Barrier Coating. The night guard was then finished and polished.

### Example 16. Laminated Night Guard Cured Without a Die Silicone

A wedge-shaped laminated arch form with an arch Parafilm backing prepared by following the procedure of Example 10 was applied over dentition in a patient's mouth. The composition was shaped using finger pressure and trimming to form a night guard. After the night guard was examined and adjusted to fit inside the mouth, it was partially-cured in the mouth for 2 to 3 minutes using a handheld light (sold by Dentsply International under the trademark: QHL75®). The partially-cured night guard was then removed from the mouth. Air barrier coating (sold by Dentsply International under the trademark: Eclipse®) was painted onto both tissue side and occlusal surface side of polymerizable night guard and then was cured for 6 minutes in a light-curing unit (sold by Dentsply International under the trademark: Eclipse®) or 10 minutes in Enterra® (sold by Dentsply International). Parafilm residue was removed from the cured night guard as needed. Finally, it was washed with water to remove all traces of Air Barrier Coating. The night guard was then finished and polished.

### Example 17. Tackiness of Polymerizable Material

In this Example, the polymerizable material of Example 4 was cured and ground to form particulate powder. The powder was added to the polymerizable material as shown in Example 5 (in an amount of 11 wt.%), and this material of Example 5 was tested for tackiness according to the test methods in ASTM D3121-06 (modified) as described above. The polymerizable material exhibited a ball tack of greater than 101.6 mm (4 inches) at 37°C.

### Example 17A. Tackiness of Polymerizable Material

In this Example, the polymerizable material of Example 4 was tested for tackiness according to the test methods in ASTM D3121-06 (modified) as described above. The polymerizable material did not contain any particulate powder. The polymerizable material exhibited a ball tack of less than 101.6 mm (4 inches) at 37°C.

As shown in Examples 17 and 17A, polymerizable materials, which contain particulate polymeric powder, show reduced tackiness. The material in Example 17 is less sticky and tacky than the material in Example 17A. In addition, the polymerizable material in Example 17 has surprisingly improved handling properties and shows increased shape-stability at elevated temperatures. These materials can be molded easily in the mouth to form a dental guard having good dimensional-stability. The materials do not flow excessively, and they can be molded and contoured to the desired shape.

Prior light-curable dental guards include those made from polymerizable compositions, such as Triad TranSheet and Eclipse Clear Baseplate, sold by Dentsply International Inc. As shown in Table 1, at 23°C, the flexural strength and flexural modulus for a dental guard material prepared by following the procedure of Example 5 is less than one-half of the flexural strength and flexural modulus for dental guards made from Eclipse Clear Baseplate and Triad TranSheet. For any particular shape (or geometry), flexural modulus is representative of the force required to deform (or deflect) a material.

**Table 1.**

| Light-curable Dental Guard materials | Flexural strength at 37°C (psi) | Flexural strength at 23°C (psi) | Flexural modulus at 37°C (kpsi) | Flexural modulus at 23°C (kpsi) | Un-notched Izod impact at 23°C (ft/lbs/in²) |
|---|---|---|---|---|---|
| Example 5 | (1,040) 7.17 MPa | (5.500) 37.9 MPa | (37) 255.1 MPa | (171) 1.18 GPa | 8.4 17.7kJ/m² |
| TRIAD TranSheet | (11,000) 75.8 MPa | (12,500) 86.2 MPa | (330) 2.27 GPa | (360) 2.48 GPa | 2.1 4.4 kJ/m² |
| ECLIPSE Clear Baseplate | (14,100) 97.2 MPa | (16,400) 113.1 MPa | (366) 2.52 GPa | (432) 2.98 GPa | 9.2 19.3 kJ/m² |
| Partially-cured Example 5 | | (570) 3.9 MPa | | (12.6) 86.9 MPa | |

As shown in Table 1, at 37°C, the flexural strength of dental guards made from Eclipse Clear Baseplate and Triad TranSheet is more than ten times the flexural strength of dental guard materials prepared by following the procedures of Example 5. Also, as shown in Table 1, at 37°C the flexural modulus of dental guards made from Eclipse Clear Baseplate and Triad TranSheet is about ten times the flexural modulus of dental guard materials prepared by following the procedures of Example 5. There is even lower flexural strength and modulus for partially-cured dental guards of this invention as shown in the last row of Table 1 (partially-cured / Example 5). The partially-cured dental guards are sufficiently dimensionally-stable under limited finger pressure and maintain some shape memory without deformation The low flexural strength and low flexural modulus of dental guards made in accordance with the invention enable them to be at least partially-cured in the mouth and then removed from the patient's mouth easily without hurting the patient. It is beneficial to have dental guards with relatively low flexural strength and modulus for comfort, easy insertion and removal. For the laminated dental guard, the flexural strength and flexural modulus of the hard layer can be higher since the soft layer provides the additional comfort, easy insertion, and removal.

It should be understood that while the present invention has been described in considerable detail with respect to certain specific embodiments thereof, it should not be considered limited to such embodiments but may be used in other ways without departing from the scope of the appended claims.

## Claims

1. A kit for making a dental guard, comprising:
i. a dental guard (28) having a U-shaped base with opposing upper and lower planar surfaces, an inner surface (29) and outer surface (31), the base comprising a material that is adapted to be molded to conform to a shape of a dental arch of a patient and cured by light irradiation to retain said molded shape, the base material comprising at least a polymerizable acrylic compound, and polymerization system adapted to be activated by light; **characterised by**
ii. a flexible tray (10) having upper and lower planar surfaces, the tray having a recessed U-shaped portion in the upper surface for holding the U-shaped dental guard; and
iii. a U-shaped backing film (30) overlying the U-shaped dental guard.

2. The kit of claim 1, wherein the flexible tray comprises a material selected from the group consisting of silicones, rubbers, and elastomeric materials.

3. The kit of claim 1, wherein the kit further comprises a release liner (32) for placing over the U-shaped dental guard and backing film.

4. The kit of claim 3 comprising the U-shaped dental guard, flexible tray, U-shaped backing film (30), and release liner (32), wherein the kit is vacuum sealed in a light-protective package.

5. The kit of claim 1, wherein the U-shaped backing film is a hydrocarbon wax film.

6. The kit of claim 1, wherein the base material comprises a relatively soft first layer and relatively hard second layer, the second layer overlying the first layer, the first layer comprising at least 30% by weight of aliphatic polyester urethane methacrylate and the second layer comprising less than 30% by weight of aliphatic polyester urethane methacrylate.

7. The kit of claim 1 or 6, wherein the base material further comprises a color-changing cure indicator, preferably wherein the cure indicator comprises material selected from methylene blue, Toluidine Blue, Rose Bengal, 4-[(octyloxy)phenyl]phenyl iodonium hexafluoroantimonate (OPPI), diphenyliodonium hexafluorophosphate (HPIHFP).

8. The kit of claim 6, wherein the base material further comprises particulate filler, preferably wherein:
i, the particulate filler is selected from the group consisting of inorganic and organic filler materials; or
ii, the particulate filler is an inorganic filler material selected from the group consisting of silica, alumina, titanium dioxide, iron oxide, silicon nitride, and glasses; or
iii, the particulate filler is organic filler selected from the group consisting of polymerized acrylic compounds, polyurethanes, polyolefins, polycarbonates, and polyepoxides; or
iv, the particulate filler comprises a blend of polymerized acrylic compounds which have been ground to a particle size of less than 1000 microns.

9. The kit of claim 6, wherein the base material is shape-stable at room temperature.

10. The kit of claim 6, wherein the base material defined by claim 1 or the material of the second layer defined by claim 6 comprises a semi-crystalline polymerizable acrylic compound selected from the group consisting of TBDMA; CAP-SMA; G4256; SR348; and ODA; and blends thereof; or the material of the first layer defined by claim 6 comprises a semi-crystalline polymerizable acrylic compound selected from the group consisting of TBDMA; CAP-SMA; G4256; and CN962; and blends thereof.

11. The kit of claim 1 or 6, wherein the polymerization system comprises a photoactive agent selected from the group consisting of camphorquinone; 2,4,6-trimethylbenzoyldiphenyl phosphine oxide; and ethyl (4-N,N-dimethylamino) benzoate.

12. A method of forming a dental guard, comprising the steps of:
i. providing the kit of any one claims 1 to 11;
ii. placing the dental guard in the mouth of a patient so the dental guard is molded over the patient's upper or lower dental arch;
iii. irradiating the dental guard with light while it is positioned inside of the mouth in a first light-curing step so the dental guard partially cures; and
iv. removing the partially-cured dental guard from the mouth and irradiating it with light in a second light-curing step so the dental guard fully cures.

13. The method of claim 12, wherein the dental guard is irradiated with blue visible light having a wavelength in the range of about 400 to about 500 nm in the first and second light-curing steps.

## Patentansprüche

1. Besteck zur Herstellung einer Zahnschutzvorrichtung, umfassend:
i. eine Zahnschutzvorrichtung (28) mit einer U-förmigen Basis mit gegenüberliegender oberer und unterer planarer Oberfläche, einer Innenfläche (29) und Außenfläche (31), wobei die Basis ein Material umfasst, das angepasst ist, um geformt zu werden, um mit der Form des Zahnbogens eines Patienten übereinzustimmen und durch Lichteinstrahlung ausgehärtet zu werden, um die geformte Form beizubehalten, wobei das Basismaterial mindestens eine polymerisierbare Acrylverbindung umfasst sowie ein Polymerisationssystem, das mit Licht aktiviert werden kann, **gekennzeichnet durch**
ii. einen flexiblen Löffel (10) mit oberen und unteren planaren Oberflächen, wobei der Löffel einen zurückgesetzten U-förmigen Bereich in der oberen Oberfläche zum Halten der U-förmigen Zahnschutzvorrichtung aufweist; und
iii. eine U-förmige Stützfolie (30), die die U-förmige Zahnschutzvorrichtung abdeckt.

2. Das Besteck nach Anspruch 1, wobei der flexible Löffel ein Material aus der Gruppe, bestehend aus Silikonen, Kautschuke und elastomeren Materialien umfasst.

3. Das Besteck nach Anspruch 1, wobei das Besteck ferner ein Trennpapier (32) zum Anordnen über die U-förmige Zahnschutzvorrichtung und die Stützfolie umfasst.

4. Das Besteck nach Anspruch 3, umfassend die U-förmige Zahnschutzvorrichtung, einen flexiblen Löffel, eine U-förmige Stützfolie (30), und ein Trennpapier (32), wobei das Besteck in einer lichtgeschützten Verpackung vakuumverpackt vorliegt.

5. Das Besteck nach Anspruch 1, wobei die U-förmige Stützfolie eine Kohlenwasserstoffwachs-Folie ist.

6. Das Besteck nach Anspruch 1, wobei das Basismaterial eine relativ weiche erste Schicht und eine relativ harte zweite Schicht umfasst, wobei die zweite Schicht die erste Schicht abdeckt, wobei die erste Schicht mindestens 30 Gewichts-% eines aliphatischen Polyesterurethanmethacrylats umfasst und die zweite Schicht weniger als 30 Gewichts-% eines aliphatischen Polyesterurethanmethacrylats umfasst.

7. Das Besteck nach Anspruch 1 oder 6, wobei das Basismaterial ferner einen farbveränderlichen Aushärtungsindikator umfasst, vorzugsweise, wobei der Aushärtungsindikator ein Material, ausgewählt aus Methylen-Blau, Toluidin-Blau, Bengal-Rot, 4-[(Octyloxy)phenyl]phenyliodoniumhexafluoroantimonat (OPPI), Diphenyliodoniumhexafluorophosphat (HPIHP) umfasst.

8. Das Besteck nach Anspruch 6, wobei das Basismaterial ferner einen teilchenförmigen Füllstoff umfasst, vorzugsweise, wobei:
i, der teilchenförmige Füllstoff ausgewählt ist aus der Gruppe, bestehend aus anorganischen und organischen Füllstoffmaterialien; oder
ii, der teilchenförmige Füllstoff ein anorganisches Füllstoffmaterial ist, ausgewählt aus der Gruppe, bestehend aus Silica, Aluminiumoxid, Titanoxid, Eisenoxid, Siliziumnitrid und Gläsern; oder
iii, der teilchenförmige Füllstoff ein organischer Füllstoff ist, ausgewählt aus der Gruppe, bestehend aus polymerisierten Acrylverbindungen, Polyurethanen, Polyolefinen, Polycarbonaten und Polyepoxiden; oder
iv, der teilchenförmige Füllstoff umfasst eine Mischung einer polymerisierten Acrylverbindung, die gemahlen wurde zu einer Teilchengröße von weniger als 1.000 Mikrometer.

9. Das Besteck nach Anspruch 6, wobei das Basismaterial bei Raumtemperatur formstabil ist.

10. Das Besteck nach Anspruch 6, wobei das Basismaterial, das von Anspruch 1 definiert wird oder das Material der zweiten Schicht, die in Anspruch 6 definiert wird, eine halbkristalline polymerisierbare Acrylverbindung umfasst, ausgewählt aus der Gruppe, bestehend aus TBDMA; CAP-SMA; G4256; SR348; und ODA; sowie Mischungen davon; oder wobei das Material der ersten Schicht, die in Anspruch 6 definiert wird, eine halbkristalline polymerisierbare Acrylverbindung umfasst, ausgewählt aus der Gruppe, bestehend aus TBDMA; CAP-SMA; G4256; und CN962; sowie Mischungen davon.

11. Das Besteck nach Anspruch 1 oder 6, wobei das Polymerisationssystem ein photoaktives Mittel umfasst, ausgewählt aus der Gruppe, bestehend aus Kampferchinon; 2,4,6-Trimethylbenzoyldiphenylphosphinoxid; und Ethyl(4-N,N-dimethylamino)benzoat.

12. Verfahren zur Herstellung einer Zahnschutzvorrichtung, umfassend die Schritte:
i. Bereitstellung des Bestecks nach einem der Ansprüche 1 bis 11;
ii. Anordnung der Zahnschutzvorrichtung in den Mund eines Patienten sodass die Zahnschutzvorrichtung mit dem oberen oder unteren Zahnbogen des Patienten geformt wird;
iii. Bestrahlung der Zahnschutzvorrichtung mit Licht in einem ersten Lichthärtungsschritt, während diese im Mund angeordnet ist, sodass die Zahnschutzvorrichtung teilweise aushärtet; und
iv. Entfernen der teilweise ausgehärteten Zahnschutzvorrichtung aus dem Mund und Bestrahlung derselben mit Licht in einem zweiten Lichthärtungsschritt, sodass die Zahnschutzvorrichtung vollständig aushärtet.

13. Das Verfahren nach Anspruch 12, wobei die Zahnschutzvorrichtung in den ersten und zweiten Lichthärtungsschritten mit blauem sichtbarem Licht einer Wellenlänge im Bereich von etwa 400 bis etwa 500 nm bestrahlt wird.

## Revendications

1. Kit pour la préparation d'une protection dentaire, comprenant :
i. une protection dentaire (28) ayant une base en forme de U avec des surfaces planes supérieure et inférieure opposées, et une surface intérieure (29) et une surface extérieure (31), la base comprenant une matière qui est adaptée à être moulée pour épouser une forme d'une arcade dentaire d'un patient et être durcie par photo-irradiation pour conserver ladite forme moulée, la matière de base comprenant au moins un composé acrylique polymérisable, et un système de polymérisation adapté à être activé par la lumière ; **caractérisé par** :
ii. un plateau flexible (10) ayant des surfaces planes supérieure et inférieure, le plateau ayant une portion évidée en forme de U dans la surface supérieure pour maintenir la protection dentaire en forme de U ; et
iii. un film de renforcement en forme de U (30) recouvrant la protection dentaire en forme de U.

2. Kit suivant la revendication 1, dans lequel le plateau flexible comprend une matière choisie dans le groupe consistant en des silicones, des caoutchoucs et des matières élastomères.

3. Kit suivant la revendication 1, ledit kit comprenant en outre une doublure de séparation (32) pour la mise en place sur la protection dentaire en forme de U et le film de renforcement.

4. Kit suivant la revendication 3, comprenant la protection dentaire en forme de U, le plateau flexible, le film de renforcement en forme de U (30) et la doublure de séparation (32), ledit kit étant conditionné hermétiquement sous vide dans un emballage de protection contre la lumière.

5. Kit suivant la revendication 1, dans lequel le film de renforcement en forme de U est un film de cire hydrocarbonée.

6. Kit suivant la revendication 1, dans lequel la matière de base comprend une première couche relativement souple et une seconde couche relativement dure, la seconde couche recouvrant la première couche, la première couche comprenant au moins 30 % en poids d'un polyester aliphatique-méthacrylate d'uréthane et la seconde couche comprenant moins de 30 % en poids de polyester aliphatique-méthacrylate d'uréthanne.

7. Kit suivant la revendication 1 ou 6, dans lequel la matière de base comprend en outre un indicateur de durcissement par changement de couleur, de préférence dans lequel l'indicateur de durcissement comprend une matière choisie entre le bleu de méthylène, le bleu de toluidine, le rose Bengale, l'hexafluoroantimoniate de 4-[(octyloxy)-phényl]phényliodonium (OPPI) et l'hexafluorophosphate de diphényliodonium (HPIHFP).

8. Kit suivant la revendication 6, dans lequel la matière de base comprend en outre une charge en particules, de préférence dans lequel :
i. la charge en particules est choisie dans le groupe consistant en des matières de charge inorganiques et organiques ; ou
ii. la charge en particules est une matière de charge inorganique choisie dans le groupe consistant en la silice, l'alumine, le dioxyde de titane, l'oxyde de fer, le nitrure de silicium et des verres ; ou
iii. la charge en particules est une charge organique choisie dans le groupe consistant en des composés acryliques polymérisés, des polyuréthannes, des polyoléfines, des polycarbonates et des polyépoxydes ; ou
iv. la charge en particules comprend un mélange de composés acryliques polymérisés qui ont été broyés à un diamètre de particules inférieur à 1000 micromètres.

9. Kit suivant la revendication 6, dans lequel la matière de base est douée de stabilité de forme à température ambiante.

10. Kit suivant la revendication 6, dans lequel la matière de base définie par la revendication 1, ou la matière de la seconde couche définie par la revendication 6, comprend un composé acrylique polymérisable semi-cristallin choisi dans le groupe consistant en TBDMA, CAP-SMA, G4256, SR348, et ODA, ainsi que leurs mélanges ; ou bien la matière de la première couche définie par la revendication 6 comprend un composé acrylique polymérisable semi-cristallin choisi dans le groupe consistant en TBDMA, CAP-SMA, G4256, et CN962, et leurs mélanges.

11. Kit suivant la revendication 1 ou 6, dans lequel le système de polymérisation comprend un agent photoactif choisi dans le groupe consistant en la camphoquinone, l'oxyde de 2,4,6-triméthylbenzoyldiphénylphosphine ; et le (4-N,N-diméthylamino)benzoate d'éthyle.

12. Procédé pour former une protection dentaire, comprenant les étapes de :
i. fourniture du kit de l'une quelconque des revendications 1 à 11 ;
ii. mise en place de la protection dentaire dans la bouche d'un patient de telle sorte que la protection dentaire soit moulée sur l'arcade dentaire supérieure ou inférieure du patient ;
iii. irradiation de la protection dentaire avec de la lumière tandis qu'elle est positionnée à l'intérieure de la bouche dans une première étape de photodurcissement de telle sorte que la protection dentaire durcisse partiellement ; et
iv. retrait de la protection dentaire partiellement durcie de la bouche et irradiation de cette protection dentaire avec de la lumière dans une seconde étape de photodurcissement de telle sorte que la protection dentaire durcisse totalement.

13. Procédé suivant la revendication 12, dans lequel la protection dentaire est irradiée avec de la lumière visible bleue ayant une longueur d'onde dans l'intervalle d'environ 400 à environ 500 nm dans les première et seconde étapes de photodurcissement.
